# EUROPEAN PATENT APPLICATION

(11) **EP 1 250 924 A1**
(43) Date of publication of application: **23.10.2002**
(21) Application number: 01946788.5
(22) Date of filing: 24.01.2001
(51) Int. Cl.: A61K 31/192, A61K 9/08, A61K 9/10, A61K 47/02, A61K 47/12, A61K 47/26, A61P 27/14

(54) **EYE DROPS**

(30) Priority: 26.01.2000 JP 2000017403
(71) Applicant: KYORIN PHARMACEUTICAL CO., LTD., Chiyoda-ku, Tokyo 101-0062 (JP)
(72) Inventor: KODAIRA, Hiromichi, Tochigi 323-0028 (JP); KOZUKA, Hitoshi, Oyama-shi, Tochigi 323-0820 (JP)
(74) Representative: ter Meer, Nicolaus, Dipl.-Chem., Dr.
(86) International application number: JP0100430
(87) International publication number: WO01054684

(57) **Abstract**

The invention provides a drug with potent and selective leukotriene antagonist activity as eye drops.

Aqueous and suspension eye drops with excellent stability having 4-[6-acetyl-3-[3-[(4-acetyl-3-hydroxy-2-propylphenyl)thio]propoxy]-2-propylphenoxy]butyric acid as an active ingredient have been developed.

## Description

### Technical field

The present invention relates to eye drops containing a drug 4-[6-acetyl-3-[3-[(4-acetyl-3-hydroxy-2-propylphenyl)thio]propoxy]-2-propylphenoxy]butyric acid (hereinafter abbreviated as KCA-757), having potent and selective leukotriene antagonist activity and being effective for the therapy of allergic conjunctivitis etc., as an ophthalmic agent.

### Background technologies

KCA-757 is a phenoxybutyric acid derivative, and it is found that it is a slightly soluble compound with potent and selective leukotriene antagonist activity and inhibitory function on respiratory anaphylaxis (Japanese Unexamined Patent Publication No. Hei 2-1459). The drugs which exert antagonistic effect on leukotrienes are promising in treatment of allergic diseases. For clinically applying KCA-757 to the ophthalmic region, the development of stable aqueous eye drops and stable suspension eye drops with good dispersibility having KCA-757 as an active ingredient has been desired.

When making a drug using KCA-757 as eye drops, there have been such problems in the aspect of stability that simple preparation of solution is liable to cause the precipitation of crystals on standing, because of its slight solubility, that, even if preparing as a suspension, the growth of particles is seen on standing (the particle size becomes large). The subject of the invention is to provide stable aqueous eye drops and stable suspension eye drops with good dispersibility having KCA-757 as an active ingredient.

### Disclosure of the invention

As a result of diligent investigations, in which, upon clinically applying KCA-757 eye drops, selection of different drug carriers, testing, etc. were repeated to solve the problems as described above, the inventors have been able to prepare aqueous eye drops with good stability and suspension eye drops with good stability and excellent dispersibility, leading to the completion of the invention.

The invention relates to eye drops prepared from KCA-757 and drug carriers (solvent, buffer, isotonization agent, preservative, pH adjusting agent and solubilizer or dispersing agent). At the time of preparing aqueous eye drops, powdery KCA-757 is dissolved into an alkali solution and, buffer, isotonization agent and preservative are added and dissolved. Then, pH is controlled to obtain aqueous eye drops. Moreover, at the time of preparing suspension eye drops, buffer, isotonization agent, dispersing agent and preservative are added to purified water and dissolved. Then, powdery KCA-757 is added to the solution after pH control and suspended to obtain suspension eye drops.

The buffer in the invention is selected from potassium dihydrogenphosphate, sodium hydrogenphosphate, boric acid, sodium borate, sodium citrate, sodium acetate, etc., and one or two or more kinds can be used appropriately. Among these buffers, potassium dihydrogenphosphate and sodium hydrogenphosphate are preferable in particular.

The isotonization agent in the invention is selected from sodium chloride, glycerin, glucose, etc., and one or two or more kinds can be used appropriately. Among these isotonization agents, sodium chloride and glycerin are preferable in particular.

The preservative in the invention is selected from benzalkonium chloride, benzethonium chloride, chlorobutanol, benzyl alcohol, phenylethyl alcohol, paraoxybenzoic ester, disodium ethylenediaminetetraacetate, etc., and one or two or more kinds can be used appropriately. Among these preservatives, benzalkonium chloride and chlorobutanol are preferable in particular.

The dispersing agent in the invention is selected from Polysolvate 80, polyvinyl alcohol, polyvinyl pyrrolidone, polyoxyethylene hydrogenated castor oil, etc., and one or two or more kinds can be used appropriately. Among these dispersing agents, Polysolvate 80 is preferable in particular.

The pH adjusting agent for the aqueous eye drops in the invention is selected from hydrochloric acid, acetic acid, citric acid, potassium dihydrogenphosphate, etc., and one or two or more kinds can be used appropriately. Among these, hydrochloric acid is preferable in particular.

The pH adjusting agent for the suspension eye drops in the invention is selected from sodium hydroxide, sodium carbonate, sodium hydrogencarbonate, sodium acetate, sodium borate, sodium hydrogenphosphate, sodium citrate, etc., and one or two or more kinds can be used appropriately. Among these, sodium hydroxide is preferable in particular.

### Best embodiment to put the invention into practice

In following, the invention will be illustrated based on examples, but the invention is not confined to these examples.

### <Example 1>

After 20mL of 0.1mol/L solution of sodium hydroxide were added to 0.5g of KCA-757 to dissolve, 50mL of purified water, 0.004g of potassium dihydrogenphosphate, 0.089g of sodium hydrogenphosphate and 0.8g of sodium chloride were added and dissolved. To this solution, 0.005g of benzalkonium chloride were added, and the solution was stirred. Then, 0.1mol/L hydrochloric acid was added to control the pH value to 8.5, and further purified water was added to make the overall volume 100mL.

### <Example 2>

After 60mL of purified water were added to 0.159g of potassium dihydrogenphosphate, 0.12g of sodium hydrogenphosphate, 0.88g of sodium chloride and 0.3g of chlorobutanol to dissolve, 0.01g of Polysolvate 80 was added, and the solution was stirred. Then, 0.1 mol/L solution of sodium hydroxide was added to control the pH value to 6.5. To this solution, 0.5g of KCA-757 were added, and, after stirring to disperse, purified water was added to make the overall volume 100mL.

### <Example 3>

After 60mL of purified water were added to 0.265g of potassium dihydrogenphosphate, 0.2g of sodium hydrogenphosphate, 0.88g of sodium chloride and 0.3g of chlorobutanol to dissolve, 0.01g of Polysolvate 80 was added, and the solution was stirred. Then, 0.1 mol/L solution of sodium hydroxide was added to control the pH value to 6.5. To this solution, 0.5g of KCA-757 were added, and, after stirring to disperse, purified water was added to make the overall volume 100mL.

### <Example 4>

After 50mL of purified water were added to 2.34g of glycerin and the solution was stirred, 0.265g of potassium dihydrogenphosphate, 0.2g of sodium hydrogenphosphate and 0.3g of chlorobutanol were added to dissolve. To this solution, 0.01g of Polysolvate 80 was added, and the solution was stirred. Then, 0.1mol /L solution of sodium hydroxide was added to control the pH value to 6.5. Further, 0.5g of KCA-757 were added, and, after stirring to disperse, purified water was added to make the overall volume 100mL.

### <Example 5>

After 50mL of purified water were added to 2.34g of glycerin and the solution was stirred, 0.53g of potassium dihydrogenphosphate, 0.4g of sodium hydrogenphosphate and 0.3g of chlorobutanol were added to dissolve. To this solution, 0.01g of Polysolvate 80 was added, and the solution was stirred. Then, 0.1mol/L solution of sodium hydroxide was added to control the pH value to 6.5. Further, 0.5g of KCA-757 were added, and, after stirring to disperse, purified water was added to make the overall volume 100mL.

### <Example 6>

After 60mL of purified water were added to 0.009g of potassium dihydrogenphosphate, 0.066g of sodium hydrogenphosphate and 0.88g of sodium chloride to dissolve, 0.01g of Polysolvate 80 and 0.005g of benzalkonium chloride were added, and the solution was stirred. Then, 0.1mol /L solution of sodium hydroxide was added to control the pH value to 7.4. To this solution, 0.3g of KCA-757 were added, and, after stirring to disperse, purified water was added to make the overall volume 100mL.

### <Test example 1>

Of the eye drops obtained in Example 1 through Example 6, the stability test was performed. Appearance was judged visually and, for quantitative determination, remaining quantity of KCA-757 was measured by means of high-performance liquid chromatography (HPLC). The particle size was measured with particle size distribution analyzer (laser-type) to calculate the 50% diameter.

The results are shown in Table 1. The stability of all drugs is good and, with respect to the particle size in suspension eye drops, drugs adapting enough to the standard of the Japanese pharmacopoeia (75µm or less) were obtained.

**(Table 1)**

| Results of the stability of KCA-757 eye drops (40°C·75%RH) | | | | | | | |
|---|---|---|---|---|---|---|---|
| Testing item Testing item | | Example 1 | Example 2 | Example 3 | Example 4 | Example 5 | Example 6 |
| Appearance | At start | Colorless, Transparent | White Homogeneous SuspenSion | White Homoge-eous SuspenSion | White HomogeNeous SuspenSion | White HomogeNeous SuspenSion | White homogeneous suspension |
| | After 1month | Same as Above | Same as Above | Same as Above | Same as Above | Same as Above | Same as above |
| PH | At start | 8.43 | 6.46 | 6.53 | 6.55 | 6.55 | 7.29 |
| | After 1 month | 8.19 | 6.10 | 6.28 | 6.22 | 6.34 | 7.29 |
| QuantitaTively deTermined Remaining Rate (%) | At start | 100 | 100 | 100 | 100 | 100 | 100 |
| | After 1 month | 99.5 | 94.1 | 97.1 | 95.2 | 95.4 | 100.9 |
| Particle Size (µm) | At start | - | 7.1 | 6.7 | 7.3 | 6.9 | 7.4 |
| | After 1 month | - | 7.7 | 7.1 | 7.8 | 7.6 | 11.9 |
| (All samples were placed in polyethylene containers for eye drops.) | | | | | | | |

### <Test example 2>

Of the eye drops obtained in Example 6, effects on the acute and chronic allergic conjunctivitis models of guinea pig were investigated.

### (Details of testing method)

### 1) Preparation of acute conjunctivitis model

To a guinea pig was administered intraperitoneally 10µg of oval-bumin (OA) together with 100mg of aluminum hydroxide gel. After 2 weeks sensitization, drops of 10µL of 2.5% aqueous solution of OA were put in both eyes. After 24 hours, drops of 10µL of 2.5% aqueous solution of OA were put in eyes again.

### 2) Effect of KCA-757 eye drops on the chemosis of acute conjunctivitis model

Drops of each 10µL of KCA-757 eye drops were put in both eyes at the times of 30, 15 and 5 minutes before the second antigenic instillation, and the chemosis after 30 minutes of antigenic instillation was observed with naked eye to score according to following criterions.
- 0 (-):: No change
- 2 (+):: Perception of slight chemosis on eyelid or conjunctiva
- 4 (++):: Perception of strong chemosis on eyelid or conjunctiva
- 6 (+++):: Perception of strong chemosis on overall eyelid or conjunctiva
Besides, when the symptom lies between respective criterions, it was expressed by 1 (±), 3 (+∼++) or 5 (++∼+++).

### 3) Effect of KCA-757 eye drops on the inframmatory cell infiltration and the injury of tissue of acute conjunctivitis model

After 12 hours of the second antigenic instillation, a blow was given on the head of guinea pig to allow to faint, and then blood was drawn, thus leading to the death. The conjunctival tissue was removed together with eyeballs, which were fixed with 10% formalin buffer. After embedded into paraffin, sectioned specimen was prepared with microtome and hematoxylin-eosin (H-E) stain was performed to observe the number of eosinophilic leukocytes (400 magnifications) and the injury of conjunctival epithelium (100∼400 magnifications) under microscope. For the inframmatory cell infiltration, number of cells at four sites in the top and bottom conjunctivae (250µm X 250µm/site, each two sites in top and bottom conjunctivae), where strong cell infiltration was perceived, was counted and expressed in terms of total number. For the injury of conjunctival epithelium, length of the injured portions in overall region of the top and bottom conjunctival epithelia was measured and expressed in terms of sum of lengths.

### 4) Preparation of chronic conjunctivitis model

To a guinea pig was administered intraperitoneally 10µg of OA together with 100mg of aluminum hydroxide gel. From the time of 2 weeks sensitization, drops of each 10µL of 2.5% aqueous solution of OA were put in both eyes eight times every fourth day.

### 5) Effect of KCA-757 eye drops on the inframmatory cell infiltration of chronic conjunctivitis model

Drops of each 10µL of KCA-757 eye drops were put in eyes each 6 times a day for 3 days from next day of the seventh antigenic instillation, and further at the times of 30, 15 and 5 minutes before and 0.5, 1, 2, 3, 4 and 5 hours after the eighth antigenic instillation. Six hours after the eighth antigenic instillation, a blow was given on the head of guinea pig to allow to faint, and blood was drawn, thus leading to the death. Then, specimen was prepared by the same method as in Testing method-3 to count the number of eosinophilic leukocytes (400 magnifications) under microscope.

The results obtained as above are shown in Table 2. Conspicuous inhibitory effects were recognized on the wide lesions over slight to heavy degree of chemosis, infiltration of eosinophilic leukocytes into conjunctival tissue, injury of conjunctival tissue, and the like having been caused in respective models.

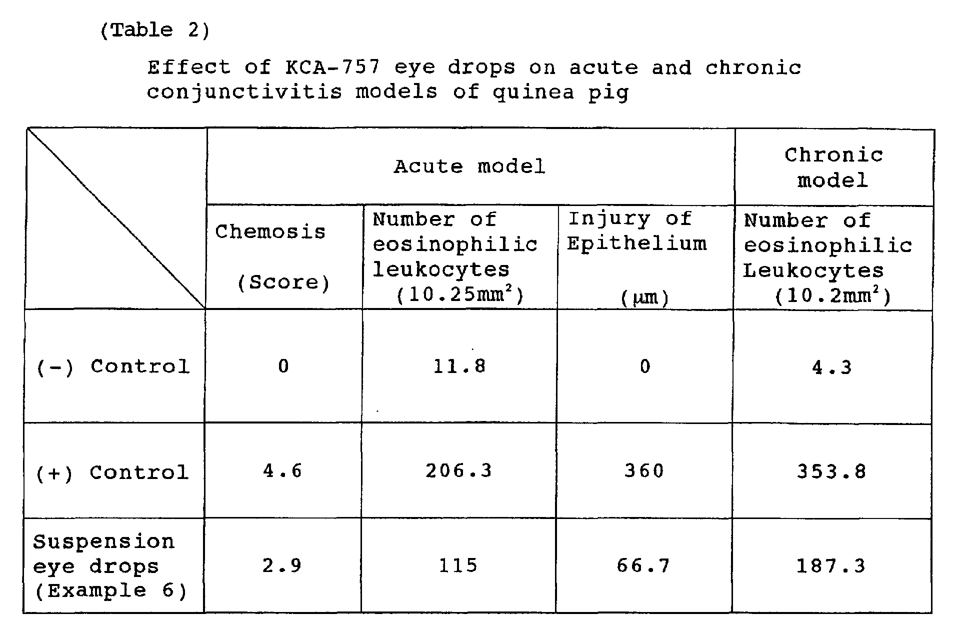

### Utilizability in the industry

The KCA-757 aqueous eye drops and suspension eye drops prepared according to the invention showed excellent stability also to the physical and chemical changes such as agglutination of active ingredient powder, change in pH and formation of decomposition products.

In addition, the inventive eye drops exhibited conspicuous inhibitory effects on the wide lesions over slight to heavy degree of chemosis, infiltration of eosinophilic leukocytes into conjunctival tissue, injury of conjunctival tissue, and the like caused in the acute and chronic allergic conjunctivitis models of guinea pig. From the facts above, the aqueous and suspension eye drops of KCA-757 with excellent stability prepared according to the invention are useful as novel therapeutic drugs for allergic ophthalmic diseases.

## Claims

1. Eye drops having 4-[6-acetyl-3-[3-[(4-acetyl-3-hydroxy-2-propylphenyl)thio]propoxy]-2-propylphenoxy]butyric acid and/or its alkali salts as active ingredient(s).

2. Aqueous eye drops having 4-[6-acetyl-3-[3-[(4-acetyl-3-hydroxy-2-propylphenyl)thio]propoxy]-2-propylphenoxy]butyric acid as an active ingredient, and comprising it and drug carriers.

3. Suspension eye drops having 4-[6-acetyl-3-[3-[(4-acetyl-3-hydroxy-2-propylphenyl)thio]propoxy]-2-propylphenoxy]butyric acid as an active ingredient, and comprising it and drug carriers.

4. The aqueous eye drops of Claim 2 containing water, alkali, buffer, isotonization agent, preservative and pH adjusting agent as drug carriers.

5. The suspension eye drops of Claim 3 containing water, buffer, isotonization agent, dispersing agent, preservative and pH adjusing agent as drug carriers.

6. The aqueous and suspension eye drops of Claim 4 or Claim 5, **characterized in that** the buffers are one or more kinds selected from phosphate, boric acid, sodium borate, sodium citrate and sodium acetate.

7. The aqueous and suspension eye drops of Claim 4 or Claim 5, **characterized in that** the isotonization agents are one or more kinds selected from sodium chloride, glycerin and glucose.

8. The aqueous and suspension eye drops of Claim 4 or Claim 5, **characterized in that** the preservatives are one or more kinds selected from benzalkonium chloride, benzethonium chloride, chlorobutanol, benzyl alcohol, phenylethyl alcohol, paraoxybenzoic ester and disodium ethylenediaminetetraacetate.

9. The suspension eye drops of Claim 5, **characterized in that** the dispersants are one or more kinds selected from Polysolvate 80, polyvinyl alcohol, polyvinyl pyrrolidone and polyoxyethylene hydrogenated castor oil.

10. The aqueous eye drops of Claim 4, **characterized in that** the pH adjusting agent are one or more kinds selected from hydrochloric acid, acetic acid, citric acid and phosphate.

11. The suspension eye drops of Claim 5, **characterized in that** the pH adjusting agent are one or more kinds selected from sodium hydroxide, sodium carbonate, sodium hydrogencarbonate, sodium acetate, sodium borate and phosphate.

12. The aqueous eye drops of Claim 4 comprising phosphate as a buffer, sodium chloride as an isotonization agent, benzalkonium chloride as a preservative and hydrochloric acid as a pH adjusting agent.

13. The suspension eye drops of Claim 5 comprising phosphate as a buffer, sodium chloride or glycerin as an isotonization agent, Polysolvate 80 or polyvinyl alcohol as a dispersing agent, benzalkonium chloride or chlorobutanol as a preservative and sodium hydroxide as a pH adjusting agent.

14. The aqueous eye drops of Claim 4 obtainable by dissolving 4-[6-acetyl-3-[3-[(4-acetyl-3-hydroxy-2-propylphenyl)thio]propoxy]-2-propylphenoxy]butyric acid into alkali solution and adding buffer, isotonization agent, preservative and pH adjusting agent.

15. The suspension eye drops of Claim 5 obtainable by adding 4-[6-acetyl-3-[3-[(4-acetyl-3-hydroxy-2-propylphenyl)thio]propoxy]-2-propylphenoxy]butyric acid to a solution of buffer, isotonization agent, dispersing agent, preservative and pH adjusting agent and dispersing it.
